Europäisches Patentamt
European Patent Office
Office européen des brevets

(11) Veröffentlichungsnummer: **0 239 867**
**A1**

(12) **EUROPÄISCHE PATENTANMELDUNG**

(21) Anmeldenummer: **87103891.5**

(22) Anmeldetag: **17.03.87**

(51) Int. Cl.⁴: **C07D 235/02**, C07F 9/65, G03F 7/10, G03C 1/72

(30) Priorität: **20.03.86 DE 3609318**

(43) Veröffentlichungstag der Anmeldung: **07.10.87 Patentblatt 87/41**

(84) Benannte Vertragsstaaten: **DE FR GB IT NL**

(71) Anmelder: **BASF Aktiengesellschaft**
**Carl-Bosch-Strasse 38**
**D-6700 Ludwigshafen(DE)**

(72) Erfinder: **Loerzer, Thomas, Dr.**
**Carl-Bosch-Ring 2**
**D-6710 Frankenthal(DE)**
Erfinder: **Leyrer, Reinhold J.,Dr.**
**Menzelstrasse 4**
**D-6700 Ludwigshafen(DE)**

(54) **Phenanthroimidazol-Verbindungen, Verfahren zu ihrer Herstellung und ihre Verwendung.**

(57) Phenanthroimidazol-Verbindungen der allgemeinen Formel (I) oder (II)

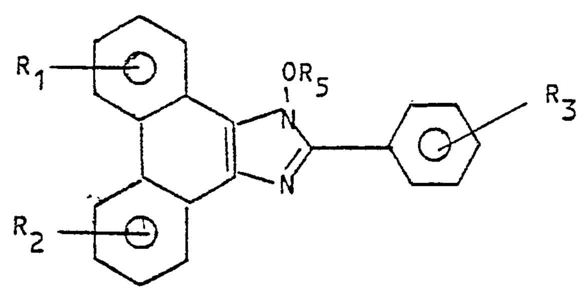

(I),

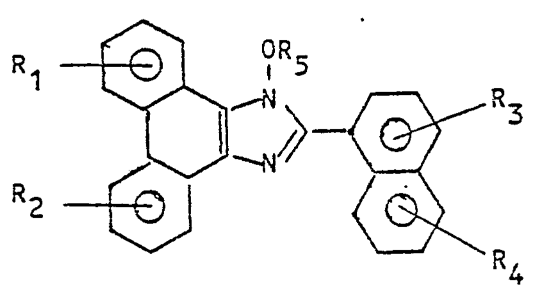

(II),

worin die Reste $R_1$ bis $R_4$ untereinander gleich oder verschieden sind und für Wasserstoffe oder Alkyl-, Alkoxy-, Halogen-oder Aminoreste stehen
und $R_5$ für einen Alkyl-, Alkoxy-oder Arylalkylrest, einen Alkylsulfonyl-, Arylsulfonyl-, Dialkoxyphosphoryl-, Dialkylphosphoryl-, Diarylphosphoryl-oder einen Carbonylrest
---$R_6$

steht, wobei $R_6$ für einen Alkyl-, Alkoxy-, Phenyl-oder Dialkylaminorest steht.

EP 0 239 867 A1

Xerox Copy Centre

Die neuen Phenanthroimidazol-Verbindungen eignen sich als Fotoinitiatoren für fotopolymerisierbare Überzugs-und Aufzeichnungsmaterialien.

## Phenanthroimidazol-Verbindungen, Verfahren zu ihrer Herstellung und ihre Verwendung

Die vorliegende Erfindung betrifft Phenanthroimidazol-Verbindungen, Verfahren zu deren Herstellung, fotopolymerisierbare Überzugs-und Aufzeichnungsmaterialien, die diese Phenanthroimidazol-Verbindungen als Fotoinitiatoren enthalten, sowie ihre Verwendung als lithographische Schicht und zur Herstellung von Druckplatten für den Hochdruck, den Tiefdruck oder die Ätztechnik.

Fotopolymerisierbare Gemische und damit hergestellte Überzugs-und Aufzeichnungsmaterialien sind zur Herstellung von Reliefdruckplatten, lithographischen Druckformen, gedruckten Schaltungen etc. verwendet worden. Im Rahmen dieser Anwendungen belichtet man diese fotopolymerisierbaren Überzugs-und Aufzeichnungsmaterialien mit aktinischem Licht und härtet so die Schicht, die üblicherweise eine Mischung aus einem polymeren Bindemittel mit mindestens einem Monomeren mit mindestens einer fotopolymerisierbaren olefinisch ungesättigten Doppelbindung darstellt.

Bei der Fotopolymerisation ethylenisch ungesättigter Verbindungen sind viele Initiatoren für die Erhöhung der Polymerisationsgeschwindigkeit bekannt. Einige dieser Initiatoren stellen 1,2-Dicarbonylverbindungen dar, beispielsweise Diacetyl, Benzil; weitere Initiatoren sind: $\alpha$-kohlenwasserstoffsubstituierte aromatische Acyloine, beispielsweise $\alpha$-Methylbenzoin; mehrkernige Chinone und eine Kombination von freie Radikale liefernde Substanzen und 2,4,5-Triphenylimidazolyl-Dimeren. Auf der Suche nach neuen Fotoinitiatoren, die eine möglichst hohe spektrale Empfindlichkeit besitzen, absolut lagerstabil, auch bei thermischer Belastung, sind sowie relativ schwerflüchtig sind, was besonders in dünnen Schichten von Bedeutung ist, wurde überraschenderweise gefunden, daß spezielle oxysubstituierte Phenanthroimidazole bei Bestrahlung mit aktinischem Licht in reaktive Spezies übergehen, die die Fotopolymerisation von ethylenisch ungesättigten Monomeren einleiten.

Gegenstand der vorliegenden Erfindung sind Phenanthroimidazol-Verbindungen der allgemeinen Formel (I) oder (II)

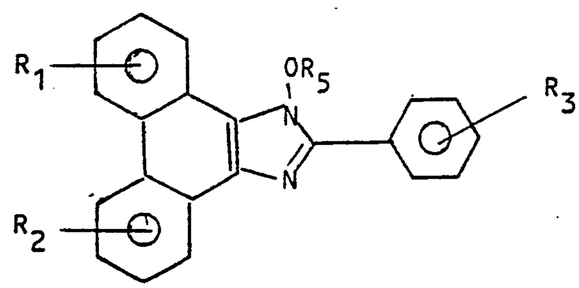

(I)

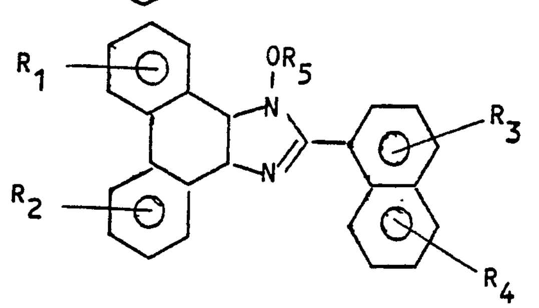

(II)

worin die Reste $R_1$ bis $R_4$ untereinander gleich oder verschieden sind und für Wasserstoff oder Alkyl-, Alkoxy-, Halogen-oder Aminoreste stehen und $R_5$ für einen Alkyl-, Alkoxy-oder Arylalkylrest, einen Alkylsulfonyl-Arylsulfonyl-, Dialkoxyphosphoryl-, Dialkylphosphoryl-, Diarylphosphoryl-oder einen Carbonylrest $-\underset{O}{C}-R_6$ steht, wobei $R_6$ für einen Alkyl-, Alkoxy-, Phenyl-oder Dialkylaminorest steht. Bevorzugt sind insbesondere solche Phenanthroimidazolverbindungen der Formel (I) oder (II), in denen $R_5$ für einen der Reste

$-C-CH_3$, $-C_2H_5$, $-C-O-CH_3$,
O O

$-C-N(CH_3)_2$, $-CN(C_4H_9)_2$, $-C-N(C_2H_5)_2$
O O O
$-C-O-C_2H_5$, $-C-O-C_6H_5$, $-P-(C_2H_5)_2$, oder
O O O
$-SO_2C_6H_4-CH_3$ steht.

Gegenstand der vorliegenden Erfindung ist auch ein Verfahren zur Herstellung dieser Phenanthroimidazol-Verbindungen, wobei das entsprechende 1-Hydroxy-2-aryl-phenanthroimidazol mit einem entsprechenden elektrophilen Reagenz umgesetzt wird.

Gegenstand der vorliegenden Erfindung sind außerdem fotopolymerisierbare Überzugs-und Aufzeichnungsmaterialien, die mindestens eine fotopolymerisierbare olefinisch ungesättigte organische Verbindung, einen Fotoinitiator sowie gegebenenfalls weitere Hilfs-und Zusatzstoffe enthalten, wobei sie als Fotoinitiator eine der erfindungsgemäßen Phenanthroimidazolverbindungen enthalten. Eine besondere Ausführungsform der Erfindung besteht darin, daß die fotopolymerisierbaren Überzugs-und Aufzeichnungsmaterialien zusätzlich ein polymeres Bindemittel enthalten.

Gegenstand der vorliegenden Erfindung ist auch die Verwendung dieser fotopolymerisierbaren Überzugs-und Aufzeichnungsmaterialien als lithographische Schicht zur Herstellung von Fotoresists oder Offset-Druckplatten, sowie zur Herstellung von Druckplatten für den Hochdruck, den Tiefdruck oder die Ätztechnik. Die erfindungsgemäßen Phenanthroimidazol-Verbindungen zeichnen sich durch sehr gute Lagerstabilität, gute Verträglichkeit mit polymeren Bindemitteln sowie Schwerflüchtigkeit aus.

Bezüglich der erfindungsgemäßen Verbindungen, deren Herstellung und Verwendung ist im einzelnen folgendes auszuführen.

Die Phenanthroimidazol-Verbindungen sind Verbindungen der allgemeinen Formel (I) oder (II)

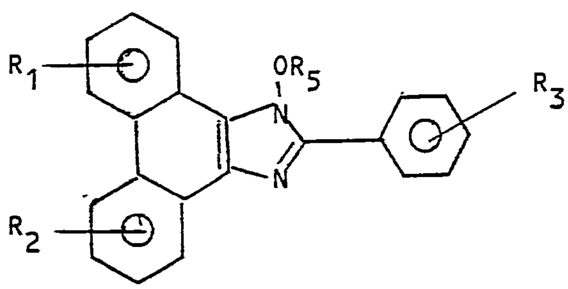

(I)

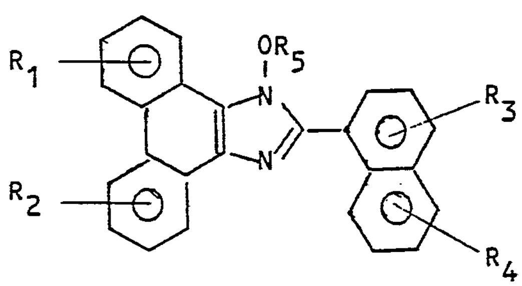

(II)

worin die Reste $R_1$ bis $R_4$ untereinander gleich oder verschieden sind und für Wasserstoff oder Alkyl-, Alkoxy-, Halogen-oder Aminoreste stehen und $R_5$ für einen Alkyl-, Alkoxy-oder Arylalkylrest, einen Alkylsulfonyl-, Arylsulfonyl-, Dialkoxyphosphoryl-, Dialkylphosphoryl-, Diarylphosphoryl oder einen Carbonyl-

rest $-\underset{O}{\underset{\|}{C}}-R_6$ steht, wobei $R_6$ für einen Alkyl-, Alkoxyl-, Phenyl-oder Dialkylaminorest steht.

Als Alkylrest $R_1$ bis $R_5$ kommen geradkettige oder verzweigte Alkylreste mit 1 bis 18, vorzugsweise 1 bis 6 Kohlenstoffatomen oder Cyclohexylreste in Frage, beispielsweise Methyl-, Äthyl-, n-Propyl, Isopropyl-, n-Butyl-, Isobutyl-, tert.-Butyl-, Pentyl-, Hexyl-, Heptyl-, Octyl-, Isooctyl-, 2-Äthylhexyl-, Decyl-, Undecyl-, Dodecyl-und Stearylreste;

als Alkoxyreste $R_1$ bis $R_5$ kommen geradkettige oder verzweigte Alkoxyreste mit 1 bis 18, vorzugsweise 1 bis 6 Kohlenstoffatomen in Betracht, beispielsweise Methoxy-, Äthoxy-, Propoxy-, Butoxy-, Pentyloxy und Hexyloxyreste;

geeignete Halogenreste $R_1$ bis $R_4$ sind Fluor, Chlor, Brom, Jod, vorzugsweise Chlor;

geeignete Aminoreste $R_1$ bis $R_4$ sind $-NH_2$, Mono-und Dialkylaminoreste mit 1 bis 6 Kohlenstoffatomen in den Alkylgruppen;

geeignete Arylalkylreste $R_5$ sind z. B. Benzylreste. Carbonylreste $R_5$ der Formel $-\underset{O}{\underset{\|}{C}}-R_6$, worin $R_6$ für einen Alkylrest mit 1 bis 18, vorzugsweise 1 bis 12 Kohlenstoffatomen, für einen Alkoxyrest mit 1 bis 18, vorzugsweise 1 bis 6 Kohlenstoffatomen, für einen Phenylrest oder einen Dialkylaminorest mit 1 bis 6 Kohlenstoffatomen in den Alkylgruppen stehen kann, sind beispielsweise Methyl, Butyl, Trimethylphenyl, Toluyl; geeignete Alkylsulfonylreste $R_5$ sind beispielsweise solche mit 1 bis 18, vorzugsweise 1 bis 6 Kohlenstoffatomen in der Alkylgruppe;

als Arylsulfonylreste $R_5$ kommen z.B. der Phenylsulfonylrest oder 4-Methylphenylsulfonylrest in Betracht;

geeignete Dialkoxyphosphorylreste $R_5$ beispielsweise solche mit 1 bis 12, vorzugsweise 1 bis 6 Kohlenstoffatomen in den Alkoxyresten;

als Dialkylphosphorylreste $R_5$ kommen z.B. solche mit 1 bis 12, vorzugsweise 1 bis 6 Kohlenstoffatomen in den Alkylgruppen in Frage;

ein geeigneter Diarylphosphorylrest $R_5$ ist z.B. der Diphenylphosphorylrest.

Beispiele für bevorzugte erfindungsgemäße Fotoinitiatoren, die diesen Formeln (I) bzw. (II) entsprechen, sind:

1-Acetoxy-2-phenylphenanthroimidazol,
1-Acetoxy-2-(2-chlorphenyl)phenanthroimidazol,
1-(N,N-Dibutylaminocarbonyloxy)-2-phenylphenanthroimidazol und
1-Benzoyloxy-2-phenylphenanthroimidazol.

Die Herstellung der jeweiligen 1-Hydroxy-2-aryl-phenanthroimidazole erfolgt nach gängigen Literaturvorschriften, so z.B. nach den Arbeiten von:

F.J. Allan, G.G. Allan, Chem. Ind. 1964, 1837;
K. Volkamer, H.W. Zimmermann, Chem. Ber. 102, 4177 (1969);
K. Akagane et al., Bull. Chem. Soc. Jap. 42, 3204 (1969).

Die Umsetzung dieser 1-Hydroxy-2-aryl-phenanthroimidazole mit elektrophilen Reagenzien erfolgt zweckmäßigerweise entweder direkt in einem geeigneten Lösungsmittel oder aber in Gegenwart basischer Verbindungen.

Die fotopolymerisierbaren Überzugs-und Aufzeichnungsmaterialien enthalten mindestens eine fotopolymerisierbare olefinisch ungesättigte organische Verbindung, vorzugsweise solche mit Siedepunkten von über 150°C, insbesondere Gemische niedrig-und höhermolekularer derartiger Verbindungen, sowie die erfindungsgemäßen Phenanthroimidazolverbindungen, im allgemeinen in Mengen von 0,1 bis 20, vorzugsweise 0,2 bis 5 Gewichts-% bezogen auf die Gesamtmenge fotopolymerisierbarer olefinisch ungesättigter organischer Verbindungen. Außerdem können sie weitere Hilfs-und Zusatzstoffe enthalten, wie z.B. Amine, insbesondere Leukofarbstoffe, beispielsweise solche aus den Gruppen

Aminotriarylmethan-Verbindungen,
Aminoxanthen-Verbindungen,
Aminothioxanthen-Verbindungen,
Amino-9,10-dihydroacridin-Verbindungen,
Aminophenoxazin-Verbindungen,
Aminophenothiazin-Verbindungen,
Aminodihydrophenazin-Verbindungen,
Aminodiphenylmethan-Verbindungen,
Leukoindamin-Verbindungen,
Aminohydrozinnsäuren,
Hydrazine,
Leukoindigoidfarbstoffe,
Amino-2,3-dihydroanthrachinone,

Tetrahalogen-p-p-biphenole,
2-(p-Oxyphenyl)-4,5-diphenylimidazole und
Phenäthylanilin-Verbindungen,
wie z.B. Kristallviolettleukobase.

Amine bzw. Lenkofarbstoffe können beispielsweise in Mengen von 0,1 bis 2 Gew.%, bezogen auf das Bindemittel, zugegeben werden.

Als polymere Bindemittel kommen insbesondere filmbildende Polymere, wie Homo-und Copolymerisate in Betracht, soweit sie die für das jeweilige Einsatzgebiet gewünschten Eigenschaften aufweisen, beispielsweise Homo-und Copolymerisate von Acrylsäure-oder Methacrylsäureestern mit 1 bis 8 Kohlenstoffatome enthaltenden Monoalkanolen, z.B. Methanol, Butanol, 2-Ethylhexanol, Copolymerisate von Vinylaromaten, wie z.B. von Styrol oder Vinyltoluol, Vinylestercopolymerisate, Vinylchlorid-und Vinylidenchloridcopolymerisate, teilweise verseiftes Polyvinylacetat, N-Vinylpyrrolidon(co)-polymerisate, Olefin/(Meth-)acrylester-Copolymerisate und Olefin/Vinylester-Copolymerisate, wobei als Olefine Äthylen, Propylen und Butadien in Frage kommen, vorzugsweise Äthylen.

Diese Copolymerisate können auch funktionnelle Gruppen, wie -COOH, und/oder -OH, $-NH_2$ durch Einpolymerisieren von Monomeren mit entsprechenden Gruppen enthalten. Als filmbildende Polymere kommen ebenso Polykondensate und Polyaddukte in Betracht, wie Polyester, Polyurethane und insbesondere Polyamide. Besonders bevorzugte filmbildende Polymere, die üblicherweise in Form ihrer Lösung auf das zu beschichtende Substrat, wie z.B. Polyester-Folie oder Aluminium-Blech aufgetragen und getrocknet werden, sind z.B. Copolymerisate von Methylmethacrylat, Acrylsäure und Hydroxypropylacrylat.

Die weiteren üblichen Zusätze variieren je nach speziellem Verwendungszweck.

Sofern es sich um fotopolymerisierbare Schichten handelt, enthalten diese fotopolymerisierbare Monomere, wie polyfunktionelle Acrylsäure-und/oder Methacrylsäureester, wie die Ester der Acryl-oder Methacrylsäure mit zwei-und mehrwertigen Alkoholen, wie Äthylenglykol, Propylenglykol, Butandiol, Hexandiol, Trimethylolpropan, Glycerin, Pentaerythrit und ähnliche, die Umsetzungsprodukte von (Poly-)Epoxidverbindungen mit (Meth-)acrylsäure, Urethanacrylate, ungesättigte Polyester, sowie Gemische der vorgenannten funktionellen Verbindungen mit monofunktionellen Verbindungen, wie Ester der Acrylsäure oder Methacrylsäure mit $C_1$-bis C 8-Monoalkanolen, Vinylestern, Vinylaromaten und/oder N-Vinyllactamen wie z.B. N-Vinylpyrrolidon.

Als weitere Zusätze sind Hilfs-und Zusatzstoffe, wie Stabilisatoren, Sensibilisatoren zu erwähnen, die im allgemeinen in untergeordneten Mengen eingesetzt werden.

Die erfindungsgemäßen fotopolymerisierbaren Überzugs-und Aufzeichnungsmaterialien können beispielsweise zur Herstellung lithographischer Schichten für Fotoresist, Offset, Hochdruck, Tiefdruck, Ätztechnik (z.B. auf Glas oder Aluminium) eingesetzt werden. Diese Verfahren sind beispielsweise in DE-OS 3 231 145, DE-OS 3 231 147, DE-OS 3 331 691, DE-OS 3 231 144 und in Günther Herrman, "Leiterplatten", Lenze-Verlag, Stuttgart, 1978 für den Fotoresist-Bereich beschrieben.

Die erfindungsgemäßen fotochromen Systeme zeichnen sich besonders durch sehr gute Lagerstabilität, Verträglichkeit mit polymeren Bindemitteln und ihre Schwerflüchtigkeit aus. Sie eignen sich besonders bei negativ arbeitenden Fotoresistfilmen.

Die im Vergleichsbeispiel und in den Beispielen genannten Teile und Prozente sind, soweit nicht anders angegeben, Gewichtsteile und Gewichtsprozente.

Beispiel 1

Herstellung von 1-Acetoxy-2(2-chlorphenyl)-phenanthroimidazol

4 Teile 1-Hydroxy-2(2-chlorphenyl)-phenanthroimidazol in 30 Teilen Tetrahydrofuran werden vorgelegt und dann mit 2 Teilen Pyridin und 3.6 Teilen Acetanhydrid versetzt. Nach fünfstündigem Rühren bei Raumtemperatur wird die entstandene Lösung mit 40 Teilen Essigester versetzt. Die so erhaltene organische Phase wird mehrfach mit Wasser gewaschen und über Natriumsulfat getrocknet. Anschließend wird das Lösungsmittel abgezogen. Der verbleibende Feststoff wird aus Methylethylketon/Cyclohexan umkristallisiert.

Man erhält 3.5 Teile eines Feststoffs mit folgenden analytischen Daten:
Schmp. 140°C
Analyse: Ber. C 71.4 H 3.9 N 7.2 Cl 9.2
Gef. C 71.3 H 4.1 N 7.2 Cl 9.2

Beispiel 2: Herstellung von 1-(N,N-Dibutylaminocarbonyloxy)-2-phenylphenanthroimidazol:

10 Teile 1-Hydroxy-2-phenylphenanthroimidazol, 8 Teile wasserfreies Kaliumcarbonat, 6,7 Teile N,N-Dibutylcarbaminsäurechlorid in 100 Teilen Tetrahydrofuran werden 5 Stunden unter Rückfluß erhitzt. Anschließend erfolgt die Zugabe von 100 Teilen Wasser und 200 Teilen Essigester. Die organische Phase wird mit Wasser gewaschen, über Natriumsulfat getrocknet und im Vacuum eingeengt. Man erhält beim Stehenlassen 6,6 Teile eines Feststoffs, der bei 120°C schmilzt und nach Dünnschichtchromatographie einheitlich ist.

Herstellung von Fotoresistfilmen:

Vergleichsbeispiel

Eine Mischung, bestehend aus 55 Teilen Polymethylmethacrylat (z.B. Degalan(R) LP 50/09, 18 Teilen Trimethylolpropantriacrylat, 13.5 Teilen Butandioldiacrylat, 9.4 Teilen p-Toluolsulfonsäureamid, 0.45 Teilen Kristallviolettleukobase, 0.13 Teilen Michler's Keton, 3 Teilen Benzophenon und 3 Teilen Hexachlorxylol in 155 Teilen Essigester, wurde hergestellt. Nach vierstündigem Rühren wurde die Lösung durch eine Druckfilter mit 1µm Porendurchmesser filtriert und auf eine 0.023 mm dicke Polyesterfolie (z.B. Melinex(R) S 23, ICI) in solcher Menge schichtförmig aufgetragen, daß nach dem Trocknen mit Warmluft ein Resistfilm von 0.035 mm Dicke verblieb.

Der Resistfilm wurde in einem üblichen Laminator (z.B. im Riston, HOT-ROLL-Laminator HRL 24) auf gebürstetes Kupfer laminiert. Der Fotolack wurde dann in einem handelsüblichen Riston B 24-Prozessor durch die Vorlage eines elektrischen Schaltbildes und des nylotron(R) -Stufenkeils 10 Takte lang belichtet. Man erhielt einen Farbumschlag zwischen belichteten und belichteten Bildteilen. Nach dem Entwickeln des Fotoresists (30 Minuten in Trichlorethan bei 18°C) stand das Bild bis nylotron-Stufenkeil 8.

Beispiel 3

Wie im Vergleichsbeispiel beschrieben wurde ein Resistfilm mit 2.5 Teilen 1-Acetoxy-2(2-chlorphenyl)-phenanthroimidazol anstelle von 0.13 Teilen Michler's Keton, 3 Teilen Benzophenon und 3 Teilen Hexachlorxylol hergestellt.

Dieser Fotolack wurde ebenfalls im Riston-B 24-Prozessor durch die Vorlage des nylotron(R)-Stufenkeils 10 Takte lang belichtet. Nach dem Entwickeln des Fotoresists (30 Minuten in Trichlorethan bei 18°C) stand das Bild bis nylotron(R)-Stufenkeil 5.

Beispiel 4

Der Fotoinitiator 1-(N,N-Dibutylaminocarbonyloxy)-2-phenylphenanthroimidazol verhält sich bei der Herstellung eines Fotoresistfilms analog Beispiel 3 ähnlich gut wie der in Beispiel 3 eingesetzte Initiator. Bei 10 Takte Belichtung erhielt man nach dem Entwickeln Stufenkeil 6.

**Ansprüche**

1. Phenanthroimidazol-Verbindungen der allgemeinen Formel (I) oder (II)

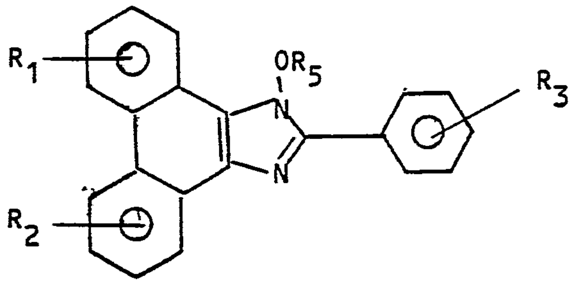

(I),

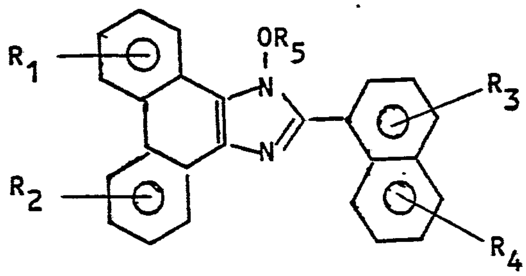

(II),

worin die Reste $R_1$ bis $R_4$ untereinander gleich oder verschieden sind und für Wasserstoffe oder Alkyl-, Alkoxy-, Halogen-oder Aminoreste stehen
und $R_5$ für einen Alkyl-, Alkoxy-oder Arylalkylrest, einen Alkylsulfonyl-, Arylsulfonyl-, Dialkoxyphosphoryl-, Dialkylphosphoryl-, Diarylphosphoryl-oder einen Carbonylrest
—$R_6$

steht, wobei $R_6$ für einen Alkyl-, Alkoxy-, Phenyl-oder Dialkylaminorest steht.

2. Verfahren zur Herstellung der Phenanthroimidazol-Verbindungen nach Anspruch 1, dadurch gekennzeichnet, daß 1-Hydroxy-2-arylphenanthroimidazole mit elektrophilen Reagenzien umgesetzt werden.

3. Fotopolymerisierbare Überzugs-und Aufzeichnungsmaterialien, die mindestens eine fotopolymerisierbare olefinisch ungesättigte organische Verbindung, einen Fotoinitiator sowie gegebenenfalls weitere Hilfs-und Zusatzstoffe enthalten, dadurch gekennzeichnet, daß sie als Fotoinitiator eine Phenanthroimidazolverbindung nach Anspruch 1 enthalten.

4. Fotopolymerisierbare Überzugs-und Aufzeichnungsmaterialien nach Anspruch 3, dadurch gekennzeichnet, daß sie zusätzlich ein polymeres Bindemittel enthalten.

5. Fotopolymerisierbare Überzugs-und Aufzeichnungsmaterialien nach Anspruch 3 oder 4, dadurch gekennzeichnet , daß als Fotoinitiator eine Phenanthroimidazol-Verbindung der allgemeinen Formel (I) oder (II) gemäß Anspruch 1 eingesetzt wird, in der $R_5$ für einen der Reste

$-\overset{\overset{O}{\|}}{C}CH_3$, $-C_2H_5$, $-\overset{\overset{O}{\|}}{C}-N(CH_3)_2$, $-\overset{\overset{O}{\|}}{C}-N(C_2H_5)_2$, $-\overset{\overset{O}{\|}}{C}-N(C_4H_9)_2$
oder $-\underset{\underset{O}{\|}}{C}-O-C_2H_5$ steht.

6. Verwendung der fotopolymerisierbaren Überzugs-und Aufzeichnungsmaterialien nach einem der Ansprüche 3, 4 oder 5 als lithographische Schicht zur Herstellung von Fotoresists oder Offset-Druckplatten.

7. Verwendung der fotopolymerisiserbaren Überzugs-und Aufzeichnungsmaterialien nach einem der Ansprüche 3, 4 oder 5 zur Herstellung von Druckplatten für den Hochdruck, den Tiefdruck oder die Ätztechnik.

Europäisches Patentamt

# EUROPÄISCHER RECHERCHENBERICHT

Nummer der Anmeldung

EP 87 10 3891

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | ANGEWANDTE CHEMIE, Band 79, Nr. 21, 7. November 1967, Seiten 941, 942, 942, Verlag Chemie, Weinheim; K. VOLKAMER et al.: "N-Oxide von Imidazolylen" * Seite 941, Verbindungen 1a, 3a * | 1 | C 07 D 235/02<br>C 07 F 9/65<br>G 03 F 7/10<br>G 03 C 1/72 |
| | --- | | |
| A | DE-C- 950 618 (KALLE & CO. AG) * Anspruch 1, Formel 26 * | 1,3,7 | |
| | --- | | |
| A | US-A-3 279 918 (P.M. CASSIERS et al.) * Tabelle 1, Verbindungen 36, 37 * | 1 | |
| | --- | | |
| A | FR-M- 5 780 (MERCK & CO., INC.) * Seite 2, Spalte 2, Zeile 45 - Seite 3, Spalte 2, Zeile 21 * | 2 | **RECHERCHIERTE SACHGEBIETE (Int. Cl.4)** |
| | --- | | C 07 D 235/00<br>C 07 F 9/00<br>G 03 F 7/00<br>G 03 C 1/00 |
| A | US-A-4 311 783 (R. DESSAUER) | | |
| | ----- | | |

Der vorliegende Recherchenbericht wurde für alle Patentansprüche erstellt.

| Recherchenort | Abschlußdatum der Recherche | Prüfer |
|---|---|---|
| BERLIN | 18-06-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82